(19) Europäisches Patentamt
European Patent Office
Office européen des brevets

(11) **EP 4 786 963 A1**

(12) **EUROPEAN PATENT APPLICATION**

(43) Date of publication:
05.08.2026 Bulletin 2026/32

(21) Application number: 25154601.6

(22) Date of filing: 29.01.2025

(51) International Patent Classification (IPC):
**G01N 24/08** (2006.01)     **G01R 33/30** (2006.01)
**G01R 33/465** (2006.01)     **C12M 3/06** (2006.01)
**G01R 33/34** (2006.01)     **G01R 33/36** (2006.01)
**G01R 33/46** (2006.01)

(52) Cooperative Patent Classification (CPC):
**G01N 24/08; C12M 23/16; G01R 33/302;
G01R 33/307; G01R 33/465;** G01R 33/34092;
G01R 33/3635; G01R 33/4608

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Danmarks Tekniske Universitet
2800 Kongens Lyngby (DK)**

(72) Inventors:
• **Wareham Mathiassen, Thomas Benjamin
2800 Kongens Lyngby (DK)**
• **Jensen, Pernille Rose
2800 Kongens Lyngby (DK)**
• **Diego Sanchez, Juan
2800 Kongens Lyngby (DK)**
• **Wang, Ke-Chuan
2800 Kongens Lyngby (DK)**

(74) Representative: **Guardian
IP Consulting I/S
Diplomvej, Building 381
2800 Kgs. Lyngby (DK)**

(54) **A CULTURE APPARATUS FOR PERFORMING NUCLEAR MAGNETIC RESONANCE SPECTROSCOPY**

(57) Various aspects disclosed herein relate to a culture apparatus for performing nuclear magnetic resonance spectroscopy, the apparatus comprising an insertion module configured to be inserted into a magnet of a system for performing nuclear magnetic resonance spectroscopy, wherein the insertion module comprises: a sample container defining a culture chamber, the culture chamber having a sample volume and being configured to contain a sample of living cells, the culture chamber being in fluidic connection with at least one supply channel for providing a fluid flow through the culture chamber, one or more radiofrequency resonators, each configured to transmit a time-varying electromagnetic wave into the sample volume of the culture chamber and/or to detect a time-varying electromagnetic wave from the sample volume of the culture chamber, wherein the sample container comprises a cell support structure defining at least one cell adherence surface configured to carry a culture of living cells within the culture chamber.

FIG. 3

EP 4 786 963 A1

**Description**

**TECHNICAL FIELD**

The present disclosure relates to a culture apparatus for performing nuclear magnetic resonance spectroscopy and to a method of performing nuclear magnetic resonance spectroscopy.

**BACKGROUND**

Nuclear Magnetic Resonance Spectroscopy (NMR) has established itself as a powerful tool for metabolic study and profiling. Uniquely, NMR is nondestructive, enabling real-time metabolic studies within living cells in solution. It is an unbiased, versatile analytical method approved for impurity quantification by authorities such as the FDA.

Organ-on-a-chip systems represent promising disease models with immense potential to revolutionize early-phase medical testing. These systems replicate the microenvironment and physiological conditions of human organs, thereby providing a versatile platform to simulate pathogen-host interactions. With their capability to mimic complex organ functions, organ chip systems hold promise for advancing drug discovery, toxicology studies, and personalized medicine.

However, to date, these systems have not been compatible with real-time metabolic imaging techniques such as NMR.

Conventional apparatus for performing NMR spectroscopy utilize a sample holder in which a small sample tube can be positioned such that the tube is surrounded by a receiver coil. The sample holder is positioned between the poles of a powerful magnet. Radio frequency radiation of appropriate energy is broadcast into the sample from an antenna coil and emission of absorbed rf energy is monitored. US 5,517,856 discloses an example of sample holder including a 5 mm glass tube.

The above prior art sample holders, where the detection electronics are tightly fitted around a 5 mm diameter glass tube, are not well suited for cell studies, such as mammalian cell studies.

WO 2023/111559 discloses a microfluidic culture apparatus comprising an insertion module configured to be inserted into a magnet of a system for performing nuclear magnetic resonance. This prior art apparatus is applicable for microfluidic perfusion culture of live tissue and is particularly applicable to the culture of tissue slices and other live systems with integrated observation of their metabolism by NMR and magnetic resonance imaging (MRI).

However, it remains desirable to provide an apparatus with an insertion module that provides sufficient stability for cell cultures to allow repeated NMR measurements and that supports long-term cultures without, or with little, cell loss.

It further remains desirable to provide a sample holder that facilitates the provision of large NMR signals from cell metabolism.

On this background, despite previous efforts, it remains desirable to provide an apparatus that solves one or more of the above problems and/or other problems, and/or that has other benefits, or that at least provides an alternative to existing solutions.

**SUMMARY**

According to one aspect, the present disclosure relates to a culture apparatus for performing nuclear magnetic resonance spectroscopy. Various embodiments of the apparatus comprise an insertion module configured to be inserted into a magnet of a system for performing nuclear magnetic resonance spectroscopy. The insertion module comprises:

- a sample container defining a culture chamber, the culture chamber having a sample volume and being configured to contain a sample of living cells, the culture chamber being in fluidic connection with at least one supply channel for providing a fluid flow through the culture chamber,

- one or more radiofrequency resonators, each configured to transmit a time-varying electromagnetic wave into the sample volume of the culture chamber and/or to detect a time-varying electromagnetic wave from the sample volume of the culture chamber.

The sample container may also be referred to as a fluidic chip or simply as a chip. In various embodiments, the sample container comprises a cell support structure defining at least one cell adherence surface configured to carry a culture of living cells within the culture chamber.

Accordingly, the cell support structure provides an adherence surface to which living cells can adhere, and the sample container facilitates maintaining perfused cell cultures of adhering cells over extended periods of time, thereby allowing metabolic analysis in a physiologically relevant environment.

In some embodiments, the cell support structure is treated, in particular coated, with a cell-adherence promoting agent such as collagen, thereby providing a tissue-like surface that improves cell adherence and viability compared to e.g. plastic surfaces.

In some embodiments, the cell support structure is a scaffold material, such as a membrane or other porous material, in particular a semipermeable membrane, e.g. a membrane having a porosity of 10% or less, such as 5% or less, such as between 1% and 10% e.g. between 1% and 5%. A collagen-treated membrane or other suitable support structure supports the attachment of adherent cells via their usual extracellular matrix proteins, thereby providing a stable cell culture that allows for repeated dDNP measurements and supports long-term cultures without cell loss.

Generally, examples of cell-adherence promoting agents include extracellular matrix proteins such as different types of collagen that may be used for different cell types, e.g. collagen types 1 to 4. Further examples include Lamanin, Fibronectin, Vitronectin, Gelatin, poly-lysine, etc.

In some embodiments, the membrane is a track-etched polycarbonate membrane. Polycarbonate membranes offer uniform pore distribution for consistent molecular transport and cell interaction, as well as optical clarity for imaging applications. Pore size can be increased or decreased to facilitate or hinder cell migration (bacterial or host). Their thin profile enables efficient diffusion of nutrients and waste, while their biocompatibility supports cell adhesion and viability, making them suitable for co-culture systems. Other membranes, like PET, also provide optical clarity and chemical resistance, while PDMS offers gas permeability and flexibility for mimicking tissue mechanics. Material choice depends on experimental needs, such as diffusion, adhesion, or structural support.

A membrane provides a large cell adherence surface area, which may extend across a major part of the cross section of the culture chamber, or even across the entire cross section of the culture chamber. In particular, the cell adherence surface may extend in a plane parallel to a primary plane defined by the culture chamber, e.g. a plane parallel to one of the side walls, in particular a largest side wall, of the culture chamber. Preferably, the cell adherence surface extends in a plane parallel to a resonator plane defined by the radiofrequency resonator, e.g. parallel to a plane defined by a planar resonator. Accordingly, the apparatus may provide a high sensitivity in the homogeneous part of the NMR spectrometer. It provides a large surface in the active detection volume, which translates into a capability of performing measurements on cultures including a large number of cells.

In some embodiments, the cell adherence surface, e.g. the membrane surface, extends across at least 20 $mm^2$, preferably more than 100 $mm^2$, preferably more than 500 $mm^2$, preferably more than 1000 $mm^2$, of the cross-sectional area of the culture chamber, thereby providing support for a large cell culture. The part of the cross-sectional area of the culture chambre across which the cell adherence surface extends will also be referred to as a cell adherence surface area. Preferably, the ratio between the sample volume and the cell adherence surface is chosen such that the sample volume can accommodate sufficient medium flow to sustain the cell culture without creating excessive sheer stress due to high flow velocities. In some embodiments, the ratio is between 0.15 mm and 1 mm, e.g. between 0.25 mm and 1 mm. Medium exchange into the culture chamber may be provided by a simple pump.

The membrane or other cell support structure may be configured to allow gas and nutrient to traverse the membrane, thereby facilitating efficient exchange of oxygen and carbon dioxide, as well as nutrients, to cells on both sides of the membrane.

The pore size of the membrane can be adapted to selectively provide a desired permeability, thereby enabling e.g. bacteria to pass through while keeping human cells separate. This is useful for infection and infiltration assays, such as evaluating barrier function by counting migrating bacteria.

The membrane or other cell support structure may be suspended or otherwise arranged in the culture chamber, e.g. directly adjacent and parallel to one of the side walls of the culture chamber or such that the cell support structure divides the culture chamber into two compartments of equal or different sizes.

When the cell support structure divides the culture chamber into two compartments, the cell support structure may define a first cell adherence surface and a second cell adherence surface, opposite the first cell adherence surface. The first cell adherence surface may face a first compartment of the two compartments, and the second cell adherence surface may face a second compartment of the two compartments, different from the first compartment.

The membrane or other cell support structure may have a predetermined permeability. A suspended, semipermeable membrane allows e.g. selective permeability, tissue differentiation and co-culturing, which is particularly interesting for bacterial infection models, T-cell migration and investigation of drug efficacy.

In some embodiments, the membrane or other cell support structure may be suspended proximal to one side wall of the culture chamber, thereby providing a single compartment on one side of the membrane.

The suspended membrane may be used to support tissue differentiation, e.g. by creating different growth environments within the portions of the culture chamber on either side of the membrane. For example, in some embodiments, one of the compartments of the culture chamber separated from each other by the membrane may be provided with one or more growth factors, thereby creating a gradient and promoting differentiation, chemotaxis, or migration. This is useful for studies involving tissue development and cell movement.

Various embodiments of the culture chamber are well-suited for studying polarized tissue, i.e. tissue that has distinct basal and apical sides. The culture chamber with a suspended membrane allows for differentiation of basal (host-facing) and apical (lumen-facing) responses. This enables the collection and analysis of respective supernatants and metabolic activities, exemplified by intestinal tissues, which receive oxygen and pyruvate from the basal side and absorb butyric acid and SCFA from the apical side.

Moreover, a suspended membrane supports the co-culturing of different cell types on either side of the membrane, thereby facilitating studies on cell-cell interactions, such as T cell migration.

In various embodiments, the culture chamber is provided with a large sample volume, in particular a sample volume of at least 200 $\mu$l, such as at least 500 $\mu$l, such as at least 700 $\mu$l. A large sample volume allows a large number of cells to be cultured while providing sufficient fluid medium to support stability over extended periods of time. The ability to perform NMR measurements on large cell cultures facilitates detection of larger NMR signals, which may improve the signal-to-noise ratio or otherwise increase the sensitivity of the measurements.

Various embodiments of the insertion module are capable of integrating existing chip assays with capabilities for proton NMR and dDNP NMR, expanding the potential for advanced research and analysis.

In some embodiments, each of the one or more radiofrequency resonators is selected from the group of radiofrequency resonators consisting of: a parallel plate resonator, a surface coil with a single turn, a surface coil with a plurality of turns, a saddle coil with one or more turns.

Here and in the following, the term saddle coil is intended to refer to a coil having a first coil portion with one or more turns arranged on a first side of the culture chamber and a second coil portion with one or more turns arranged on a second side of the culture chamber, opposite the first side. The coil portions are electrically connected with each other. Each coil portion may be planar or curved, or otherwise suitably shaped. In one embodiment, each coil portion is planar and parallel to a side wall of the sample container.

In a preferred embodiment, one or each of the one or more radiofrequency resonators is a saddle coil, e.g. forming two flat, in particular planar, coil portions opposed to each other and arranged on opposite sides of the sample container.

In another embodiment, one or each of the one or more radiofrequency resonators is a plate resonator or otherwise a resonator having only a single portion arranged on one side of the culture chamber. The use of a plate resonator may be useful if the microfluidic chip has tubings in such a way that it is not practical to have the coil arranged tightly on both sides of the chip.

The one or more radiofrequency resonators may be configured to concentrate the transmitted electromagnetic wave inside the culture chamber. Preferably, the one or more radiofrequency resonators may be configured to transmit the time-varying electromagnetic wave into the entire sample volume of the culture chamber. To this end, in some embodiments, at least one of the one or more radiofrequency resonators comprises a coil having a plurality of turns, preferably three or more turns. The plurality of turns provides a coil design that is well suited for low frequencies, in particular for detection of $^{13}$C nuclei.

In some embodiments, at least one of the one or more radiofrequency resonators comprises one or more planar resonator portions, e.g. a planar coil, a pair of planar coils or a plate resonator, thereby providing a magnetic field penetrating a large surface, which facilitates measurements across a major part, or even the entire, sample volume. In particular, in some embodiments, the culture chamber is a flat chamber having a width and a length defining a primary plane of the culture chamber, wherein the culture chamber has a height smaller than the width and the length, wherein the planar resonator portion defines a resonator plane substantially parallel to the primary plane.

Accordingly, the radiofrequency resonator may have a resonator width and a resonator length, wherein the resonator width is no smaller than the width of the culture chamber, and wherein the resonator length is no smaller than the length of the culture chamber. The resonator width and length may be defined as the width and length, respectively, of the planar resonator portion(s), e.g. of the planar coil(s) or resonator plate(s).

In some embodiments, the insertion module includes two radiofrequency resonators, e.g. radiofrequency resonators configured to have respective resonance frequencies, e.g. one radiofrequency resonator configured for detection of $^{1}$H and/or $^{2}$H nuclei and one radiofrequency resonator configured for detection of $^{13}$C nuclei or otherwise.

In some embodiments, the (or each) radiofrequency resonator may comprise two resonator portions disposed on opposite sides of the culture chamber. In particular, the (or each) radiofrequency resonator may comprise a first planar resonator portion disposed adjacent and parallel to one side wall of the culture chamber and a second planar resonator portion disposed adjacent and parallel to another, opposite side wall of the culture chamber. Each planar resonator portion may be implemented as a resonator plate, a planar coil having one or more turns, or otherwise.

In some embodiments, the cell support structure is arranged in the culture chamber with its one or more cell adherence surfaces parallel to the primary plane of the culture chamber, in particular parallel to the resonator plane, thereby providing an efficient concentration of the emitted electromagnetic wave across the cell adherence surface. For example, when the cell support structure is embodied as a membrane, the membrane may be suspended in the culture chamber parallel to the primary plane of the culture chamber, in particular parallel to the resonator plane.

In some embodiments, the one or more radiofrequency resonators are arranged adjacent to the sample container, thereby providing a compact probe and a good concentration of the transmitted electromagnetic wave inside the sample volume.

In some embodiments, the sample container is an elongated container having a first end and a second end, opposite the first end, and wherein the sample container comprises an inlet port and an outlet port, each in fluid communication with the culture chamber, wherein the inlet port and the outlet port are arranged at the first end. Accordingly, the sample container can easily be connected to conduits for supplying a flow of medium through the culture chamber, in particular of cell culture medium for sustaining the cell cells adhered to the cell support structure.

The supply channel may fluidly connect an inlet port of the sample container with an inlet onto the culture chamber thereby providing an inlet flow. In some embodiments, the supply channel has an inlet portion providing an inlet opening for providing fluid flow from the supply channel into the culture chamber, preferably such that the inlet flow is substantially parallel to the cell adherence surface. Preferably, the inlet portion is a divergent inlet portion, where the inlet opening has a cross-sectional area larger than a cross-sectional area of the supply channel, thereby reducing the flow velocity of the fluid flow into the culture chamber, which facilitates a substantially laminar fluid flow through the culture chamber across the cell adherence surface, the laminar flow being high enough to prevent oxygen depletion and/or otherwise sustain the cell culture in the culture chamber. The divergent inlet portion may have a gradually increasing cross-section, e.g. a V-shaped or conical expansion. This allows the fluid to spread out slowly as it enters the culture chamber, thereby minimizing sudden pressure changes and reducing shear stress on the cells, thereby creating a stable environment that supports cell viability and growth. The sample container may comprise a further supply channel that may fluidly connect an outlet opening of the culture chamber with an outlet port of the sample container, thereby providing a return flow of spent medium.

The present disclosure relates to different aspects, including the apparatus described above and in the following, corresponding apparatus, systems, methods, and/or products, each yielding one or more of the benefits and advantages described in connection with one or more of the other aspects, and each having one or more embodiments corresponding to the embodiments described in connection with one or more of the other aspects and/or disclosed in the appended claims.

In particular, according to one aspect, disclosed herein are embodiments of a method for performing nuclear magnetic resonance spectroscopy, the method comprising:

- providing a culture apparatus as described above and/or in the following,

- seeding the culture apparatus with cells to be analyzed and allowing the cells to adhere to the cell support structure,

- providing fluid flow of a cell culture medium through the culture chamber,

- repeatedly inserting the insertion module into a nuclear magnetic resonance instrument to obtain nuclear magnetic resonance signals from the cell culture.

**BRIEF DESCRIPRION OF THE DRAWINGS**

Embodiments of the various aspects disclosed herein will be described in more detail in connection with the appended drawings, in which:

FIG. 1 schematically illustrates an example of a system for performing nuclear magnetic resonance (NMR) spectroscopy.

FIG. 2 schematically illustrates an example of a culture apparatus for performing nuclear magnetic resonance spectroscopy.

FIG. 3 schematically illustrates an exploded view of an example of an insertion module configured to be inserted into a magnet of a system for performing nuclear magnetic resonance spectroscopy.

FIG. 4 schematically illustrates examples of a sample container.

FIGs. 5A-B schematically illustrates a more detailed view of an example of a sample container.

FIG. 6A-B schematically illustrate cross sectional views of examples of a culture chamber.

FIGs. 7A-B schematically illustrate another example of an insertion module configured to be inserted into a magnet of a system for performing nuclear magnetic resonance spectroscopy.

FIG. 8 illustrates performance of an embodiment of the insertion module.

FIG. 9 illustrates results obtained with an embodiment of the apparatus disclosed herein.

FIG. 10 shows dDNP-NMR measurement of lactate production in a confluent layer of HeLa cells in a two-compartment chamber three hours after seeding.

FIG. 11 shows Shigella specific acetate production measured during adherence to HeLa cells in one-compartment chamber.

**DETAILED DESCRIPTION**

In the following, aspects and embodiments of a culture apparatus for performing nuclear magnetic resonance spectroscopy will be described.

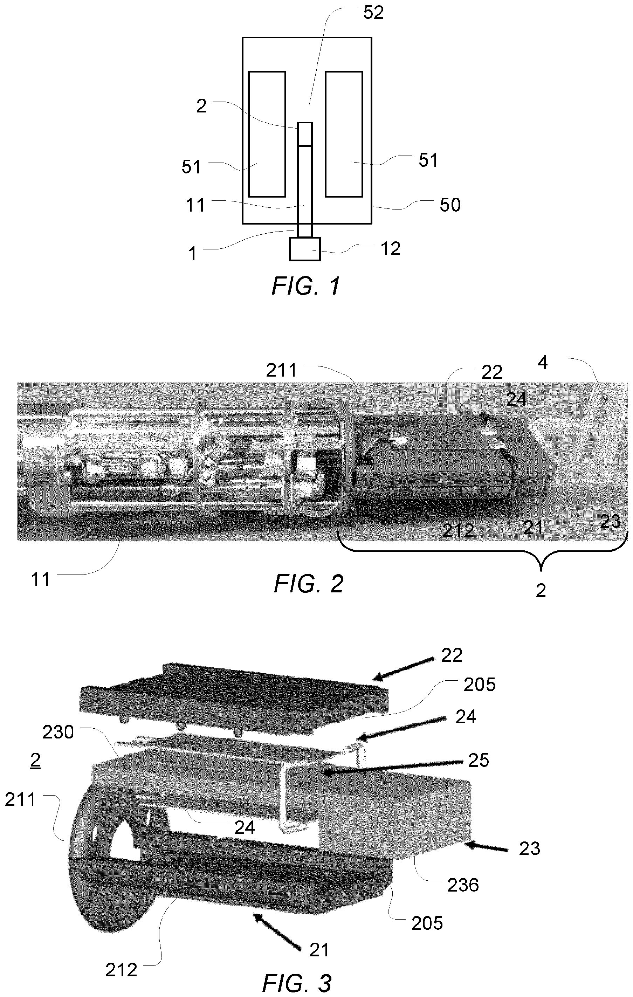

FIG. 1 schematically illustrates an example of a system for performing nuclear magnetic resonance (NMR) spectroscopy. The system is for measuring an NMR signal generated by one or more atomic nuclei contained in a sample. To this end, the system comprises a magnet assembly 50 for generating a static magnetic field. The magnet assembly 50 may include one or more magnets 51, such as cooled superconducting magnets, permanent magnets, or otherwise. The magnet assembly 50 defines a void 52 for accommodating a sample to be analyzed. The void 52 may be in the form of a bore or other form of hollow portion extending into the magnetic field generated by the magnet assembly 50. The magnetic field vector of the static magnetic field at the location of the sample within the void 52 may extend substantially axial in respect of a longitudinal axis of the void.

The system further comprises an NMR probe 1 having an insertable portion 11 configured to be inserted into the void 52. To this end, the insertable portion 11 may have a cylindrical shape 11 and be configured to be inserted into a bore of the magnet assembly. The insertable portion 11 of the NMR probe may be configured to carry an insertion module 2, e.g. accommodated at a distal end of the insertable portion 11, such that the insertion module is positionable within the magnetic field generated by the magnet assembly 50 when the insertable portion is inserted into the void 52.

The NMR probe 1 may further comprise a transmitter and/or receiver module 12, which may comprise transmitter and/or receiver circuitry connectable to one or more radiofrequency resonators accommodated in the insertable portion 11 and/or the insertion module 2. Each radiofrequency resonator may be configured for transmitting a radiofrequency electromagnetic wave and/or for detecting a magnetic response caused by the transmitted radiofrequency signal. The transmitted radiofrequency signal may have a magnetic field vector directed along a lateral direction, in particular substantially orthogonal to the magnetic field vector of the static magnetic field, or otherwise. Said one or more radiofrequency resonators may include separate resonators for transmission and detection, respectively, or one or more radiofrequency resonators for both transmitting and detecting time-varying electromagnetic waves, in particular in the radiofrequency range. Accordingly, the transmitter circuit of the transmitter and/or receiver module 12 may be configured to energize one or more radiofrequency coils for transmitting a time-varying electromagnetic wave at the position of the sample when the insertion module 2 is positioned in the void 52. Similarly, the receiver circuit may be configured to receive a detection signal from one or more radiofrequency resonators of the insertable portion 11 or of the insertion module 2.

When the insertion module 2 with a sample to be analyzed is placed in the void 52, a magnetic field is applied by the magnet assembly 50 to the sample, thus causing nuclei of the sample to align with the magnetic field. When a radiofrequency pulsatile wave is applied to the sample by the transmission circuit via a radiofrequency resonator, states of the nuclei may switch, which in turn generates a current within the same or a different radiofrequency resonator, thus obtaining a signal from the sample that is detectable by the receiver circuit. This signal may be analyzed afterwards.

FIG. 2 schematically illustrates an example of a culture apparatus for performing nuclear magnetic resonance spectroscopy. The culture apparatus comprises an insertion module 2 configured to be inserted into a magnet of a system for performing nuclear magnetic resonance spectroscopy, e.g. the system described in connection with FIG. 1 or another suitable type of NMR system. In the example of FIG. 2, the insertion module 2 is shown attached to a distal end of an insertable portion 11 of an NMR probe, e.g. of a conventional NMR probe, or otherwise.

An embodiment of the insertion module will now be described with reference to FIG. 3, which schematically illustrates an exploded view of the insertion module of FIG. 2, and with continued reference to FIG. 2.

The insertion module 2 comprises a housing, a sample container 23 and one or more radiofrequency resonators 24, 25.

The sample container 23 defines a culture chamber for accommodating a culture of living cells. The sample container 23 comprises a flat, elongated base 230 and a fluidic interface 236 positioned at one end of the elongated, flat base 230. The base 230 of the sample container comprises the culture chamber, as will be described in more detail below. The fluidic interface 236 defines one or more inlet and/or outlet ports, which are in fluid communication the culture chamber. The inlet and/or outlet ports allow the sample container 23 to be fluidly connected to one or more fluid lines 4 for supplying fluid to the culture chamber and for transporting fluid away from the culture chamber, respectively, e.g. for transporting between the sample container and one or more external fluid reservoirs, pumps, fluidic system and/or the like. The base 230 is generally planar and defines a primary plane of the culture chamber.

The one or more radiofrequency resonators 24 and 25 are each configured to transmit a time-varying electromagnetic wave into the sample volume of the culture chamber and/or to detect a time-varying electromagnetic wave from the sample volume of the culture chamber (a so-called Free Induction Decay, FID). Generally, each radiofrequency resonator may be formed as a flat resonator, e.g. plate resonator or a planar coil having a suitable number of turns. A plurality of turns may provide an improved concentration of the field close to the coil where the culture chamber is placed. In some embodiments, each resonator may have a high Q-factor (e.g. Q>50, such as Q > 100).

In the present example, the housing is provided as a two-part housing having a base part 21 and a lid part 22, thereby facilitating assembly of the insertion module. However, in other embodiments, the housing may be provided as a single part or as more than two parts that can be assembled to form the housing. The housing may be made of any suitable material, e.g. Plastic, PTFE, Kel-F, etc. The housing may be made by an additive manufacturing process, by injection molding or otherwise.

The housing comprises a mounting portion 211 and a carrier portion 212.

The mounting portion 211 is attachable to the distal end of the insertable portion 11 of the NMR probe such that the carrier portion 212 of the housing extends away from the distal end of the insertable portion of the NMR probe along the axial direction of the insertable portion 11. The mounting portion 211 may be provided as a flange at one end of the carrier portion 212, or otherwise.

The carrier portion 212 is operable as a carrier for the one or more radiofrequency resonators. To this end, the carrier portion 212 may define one or more planar support surfaces for carrying respective ones of the radiofrequency resonators 24 and 25. The carrier portion 212 further includes a void that is shaped and sized for accommodating the base 230 of the sample container sandwiched between the first and second coil portions of the coils 24 and 25. The carrier portion 212 may be shaped as a generally flat, elongated part of the housing. The carrier portion 212 may include an opening 205 allowing the sample container 23 to be selectively inserted, e.g. slidably inserted, into the housing and removed from the housing.

Both radiofrequency resonators 24 and 25 comprise respective first and second resonator portions. A first resonator portion of each resonator is disposed adjacent to a first side of the base 230 of the sample container, while a second resonator portion of each resonator is disposed adjacent a second side of the base portion, opposite the first face. The resonator portions are planar and parallel to a primary plane of the culture chamber of the sample container.

In the present example, the base part 21 of the housing is configured to carry first resonator portions of the resonators 24 and 25, while the lid part 22 is configured to carry second resonator portions of the resonators 24 and 25. To this end, the base art 21 and the lid part 22 may be provided with grooves or other features at the inward-facing and/or outward-facing walls of the lid part 22 and of the carrier portion 212 of the base portion 21, respectively.

One radiofrequency resonator 24 of the radio frequency resonators is formed as a parallel plate resonator. The parallel plate resonator includes two parallel plates, which are conductively interconnected with each other, and which are carried by the lid part 22 and the base part 21, respectively. While, in the present example, the plates of the parallel plate resonator are arranged on the outer surface of the housing, they may also be arranged on an inward facing surface of the housing.

When the sample container 23 is inserted into the housing, the base 230 of the sample container, which accommodates the culture chamber, is sandwiched between the parallel plates of the parallel plate resonator 24. The plates of the resonator 24 are parallel to the primary plane defined by the base 230 of sample container. In other examples, a pair of planar coils with one or multiple turns may be used instead of parallel plates.

The other radio frequency resonator 25 is formed as two flat coils disposed on respective inward facing walls of the carrier portion 212. The two flat coils thus also extend on opposite sides of the base 230 of the sample container. Each of the two flat coils has a single turn. However, in other examples, coils with multiple turns may be used.

In the present example, the parallel plate resonator 24 is configured to detect $^1$H and $^2$H, and the pair of flat coils 25 is configured to detect $^{13}$C.

The radiofrequency resonators may be made of a suitable electrically conductive material. The parallel plates of the parallel plate resonator 24 may be provided as copper plates or otherwise. The turns of the planar coils 25 may be made from e.g. silver or another suitable material.

Examples of sample containers will now be described in more detail below. Generally, the culture chamber has a sample volume and is configured to contain a sample of living cells. The culture chamber is connected in fluidic connection with one or more supply channels for providing a fluid flow through the culture chamber. In various embodiments, the sample volume of the culture chamber is at least 200 $\mu$l, such as at least 500 $\mu$l, such as at least 700 $\mu$l.

In various embodiments, the sample container comprises a membrane or other form of cell support structure, which may be suspended or otherwise arranged inside the culture chamber. The cell support structure is configured to carry a culture of living cells.

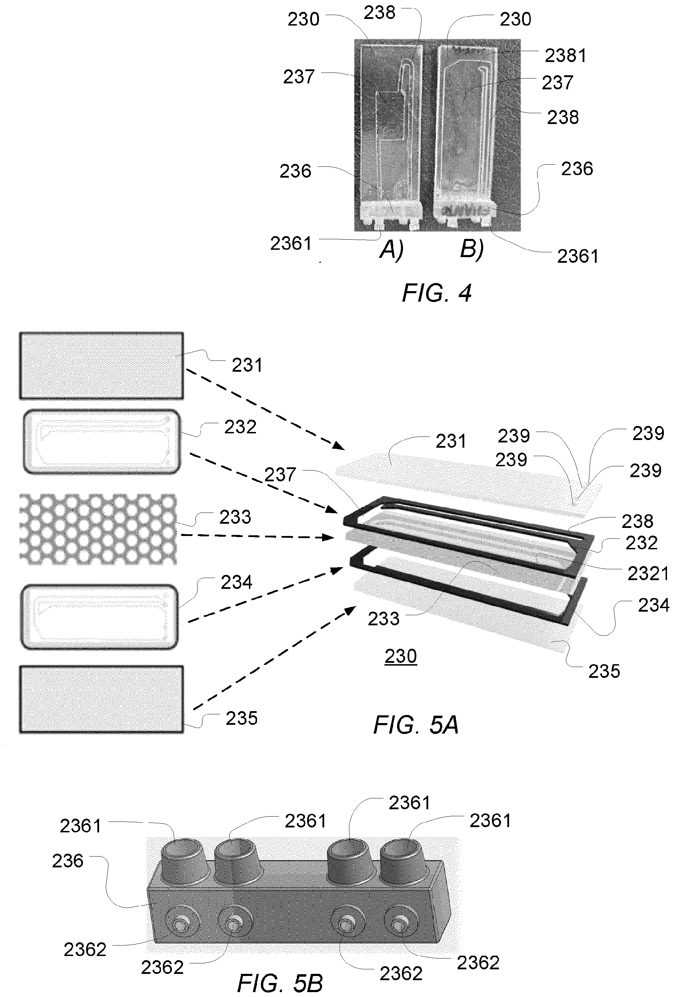

FIG. 4 schematically illustrates two examples of a sample container. The sample containers 23 of both examples may be used as part of the insertion module described in connection with FIGs. 2-3, or with another suitable insertion module. Each of the sample containers includes a flat elongated base 230 and a fluidic interface 236 positioned at one end of the elongated, flat base 230. The base 230 of the sample container comprises the culture chamber 237. The fluidic interface 236 comprises inlet and/or outlet ports 2361 which are in fluid communication with the culture chamber 237 via one or more supply channels 237 and allow the sample container to be fluidly connected to one or more fluid lines for supplying fluid to the sample container and for transporting fluid from the sample container, respectively.

In the illustrated examples, the fluidic interface comprises four ports. In some of the examples all of the ports are used as inlets or outlets to different parts of the culture chamber, in particular in examples where the chamber has different compartments separated by a membrane. In other examples, only some, e.g. only two, of the ports are used and fluidly connected to the culture chamber 237 as inlet and outlet, respectively. For example, in a culture chamber with only a single compartment it may be sufficient to provide a single inlet and a single outlet. The base 230 of the sample container may have a suitable outer dimension, e.g. 25x75 mm corresponding to a typical microscope slide size. The fluidic interface 236 may be made from biocompatible resin, e.g. by 3D printing or otherwise. The fluidic interface 236 may conveniently be made Mini Luer-compatible. The culture chamber 237 is a flat chamber having a width and a length defining a primary plane of the culture chamber, wherein the culture chamber has a height smaller than the width and the length.

The examples of FIG. 4 differ from each other by the shape and size of the culture chamber 237. In the example of FIG. 4 A), the culture chamber 237 is a generally rectangular chamber having a first end proximal to the fluidic body, and a distal end, opposite the first end. In one embodiment, the culture chamber has lateral dimensions of about 12x20 mm and a volume of about 0.3 mL. The culture chamber 237 is fluidly connected with the inlet and output ports of the fluidic interface 236 via respective supply channels 238 where one supply channel has an opening to the culture chamber at the first end of the culture chamber while the other supply channel has an opening at the second end of the culture chamber, thereby facilitating a fluid flow between the openings from one end to the opposite end of the culture chamber.

In the example of FIG. 4 B), the culture chamber 237 extends across a major part of the flat base 230 of the sample container 23. In one embodiment, the culture chamber has lateral dimensions of about 20x65 mm and a volume of about 0.8 mL. In particular, in one embodiment, the culture chamber has a single compartment having lateral dimensions of about 20x65 mm and a volume of about 0.8 mL In another embodiment, the culture chamber has lateral dimensions of about 20x65 mm and a volume of about 1 mL. In particular, in one embodiment, the culture chamber has lateral dimensions of about 20x65 mm and two compartments separated by a membrane, where the total volume of the culture chamber, i.e. the total volume of both compartments together, is between 1 mL and 1.5 mL. Of course, other embodiments may have different sizes. The culture chamber 237 has a first end proximal to the fluidic interface 236, and a distal end, opposite the first end. The culture chamber 237 is fluidly connected with the inlet and output ports 2361. In particular, one supply channel 238 extends between the fluidic interface 236 to the distal end of the culture chamber 237. The supply channel 238 may have an inlet portion 2381 that provides an opening into the culture chamber 237. The inlet portion 2381 is gradually expanding, e.g. in a v-shape or conical fashion or otherwise. This allows the fluid to spread out slowly as it enters the culture chamber 237, thereby minimizing sudden pressure changes and reducing shear stress on the cells disposed inside the culture chamber, thereby creating a stable environment that supports cell viability and growth.

FIGs. 5A-B schematically illustrate a more detailed view of an example of a sample container, e.g. the sample container of FIG. 4 B). In particular, FIG. 5A shows an exploded view of the flat, elongated base 230 of the sample container, while FIG. 5B shows a 3D view of an example of the fluidic interface 236.

The elongated, flat base 230 is formed as a layered structure comprising a plurality of layers: Two outermost layers 231 and 235, two distancer layers 232 and 234 sandwiched between the outermost layers, and a membrane layer 233 sandwiched between the distancer layers.

The distancer layers 232 and 234 may each comprise a circumferential frame 2321 defining a central cutout extending through the entire distance layer. The frame 2321 defines circumferential walls of the culture chamber 237 and of a supply channel 238 for providing a fluid connection between the fluidic interface 236 and a distal end of the culture chamber 237.

The distance layers 232 and 234 thus function as the fluidic layers that define the supply channel(s) and culture chamber. The distance layers may be made from a suitable thermoelastic material, e.g. a thermoplastic elastomer such as Styrene-Ethylene-Butadiene-Styrene (SEBS) or another suitable material. The heights of the distance layers define the height of the culture chamber. The distance layers may have the same or different heights, thus defining the relative size of the compartments of the culture chamber on either side of the membrane. In one example, one distance layer 232 has a height of about 0.75 mm, while the other distance layer 234 has a height of 0.25 mm. In an alternative embodiment, the sample container may comprise only a single distancer layer, e.g. such that the membrane is located between the distancer layer and one of the outermost layers. Accordingly, in such an embodiment, substantially the entire volume of the culture chamber is located on one side of the membrane, thus forming a single compartment. In one embodiment, the compartment defined by distance layer 232 has a volume of 0.8 mL while the compartment defined by distance layer 234 has a volume of 0.2 mL, i.e. the total volume of the two-compartment chamber is, in one embodiment, about 1 mL.

The outermost layers 231 and 235 define top and bottom walls of the culture chamber, respectively. They may be made of a suitable thermoplastic material, such as a thermoplastic polymer, e.g. PETG. The outermost layers may have different thicknesses or the same thickness. In one example, the outermost layer 231 may have a thickness of 1 mm and the other outermost layer 235 may have a thickness of 0.3 mm.

The outermost layer 231 has through holes 239 at one end providing fluid communication with the culture chamber 237 and with the supply channel 238, respectively.

The membrane 233 may be a polycarbonate membrane having a mesh structure defining pores, or it may be made from another suitable material and otherwise semipermeable. The membrane may e.g. be 10 $\mu$m

thick.

The layers may be bonded together by a suitable bonding process, e.g. a thermal bonding process. In one example, the layers are bonded at 60 °C and under a pressure of about 2 Pa, e.g. for 1 h.

The fluidic interface 236 may define a manifold of conduits connecting inlet/output ports 2361 with respective connecting ports 2362. The fluidic interface 236 is bonded to the outermost layer 231 such that the connecting ports connect to respective ones of the through holes 239. In the example of FIG. 5A, the fluidic interface 236 provides two inlets, one for each of the compartments, and two outlets, one for each compartment. It will be appreciated that the direction of the flow may be reversible, i.e. that the inlets may be operable as outlets and vice versa.

In various embodiments, the sample container 23 provides a two-compartment chamber with a porous, tissue-treated membrane in between the compartments. With a large volume of 200 $\mu$L or more, such as 700 $\mu$L or more, various embodiments are compatible with assays requiring large volumes of supernatant (e.g. 200 $\mu$L or more). For the purpose of the present description, the volume refers to the volume of the whole chamber, i.e. to the total volume of both compartments. Some embodiments of the sample container may be optimized for analyzing samples in anaerobic conditions. In alternative embodiments, the sample container may be made from oxygen-permeable plastics like PDMS.

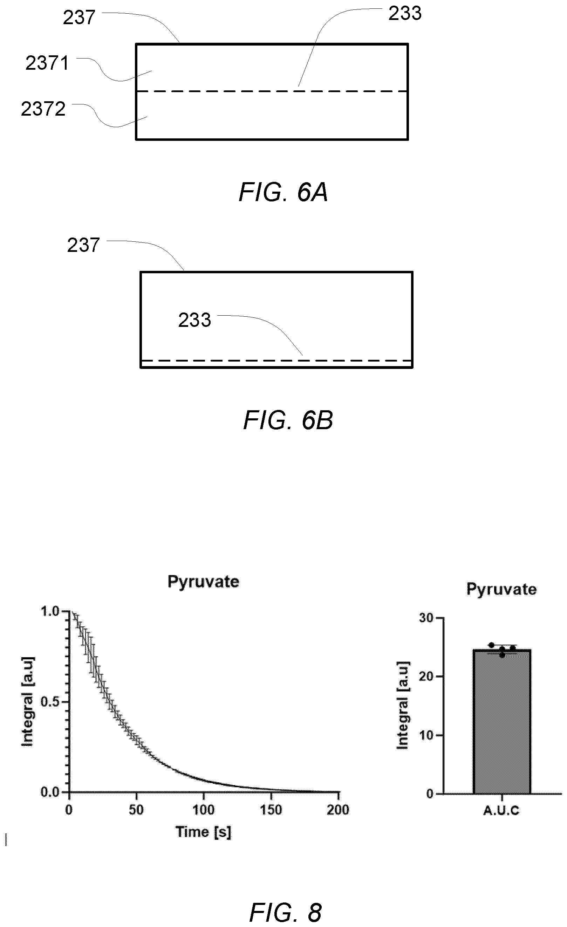

FIG. 6A-B schematically illustrate cross sectional views of examples of a culture chamber. FIG. 6A schematically illustrates a cross section of a culture chamber 237 with a suspended membrane 233 that divides the culture chamber in an upper compartment 2371 and a lower compartment 2372. FIG. 6B schematically illustrates a cross section of a culture chamber 237 with a membrane 233 disposed directly adjacent a bottom wall of the culture chamber thus resulting in a culture chamber having a single compartment.

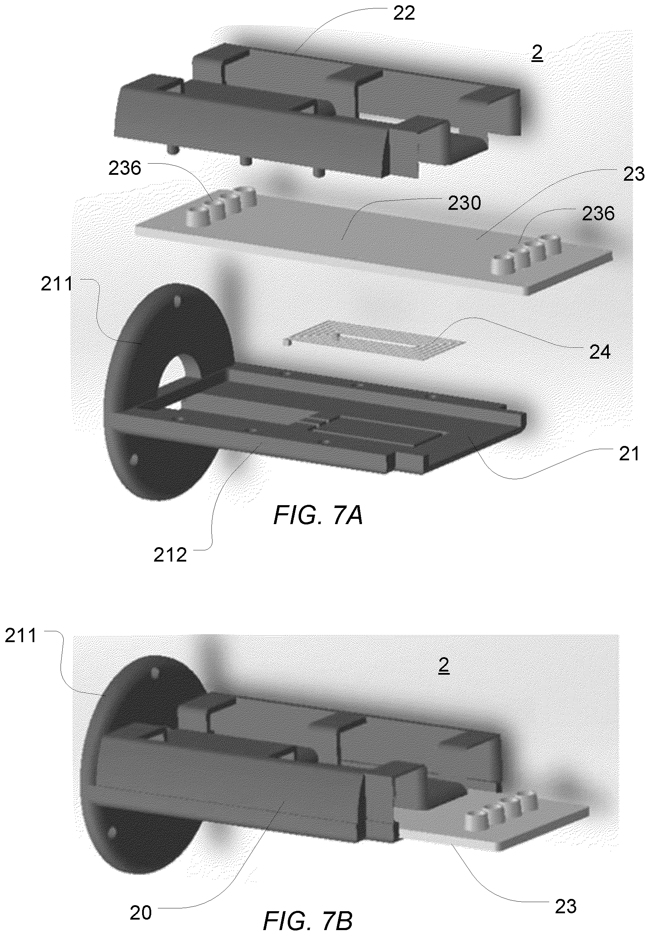

FIGs. 7A-B schematically illustrate another example of an insertion module configured to be inserted into a magnet of a system for performing nuclear magnetic resonance spectroscopy. FIG. 7A shows an exploded view of the insertion module while FIG. 7B shows a schematic view of the assembled insertion module.

The insertion module 2 of FIGs. 7A-B is similar to the embodiment of FIG. 3 in that it comprises a housing, a sample container 23 and one or more radiofrequency resonator 24. The sample container 23 comprises a flat, elongated base 230 and defines a culture chamber for accommodating a culture of living cells. The housing is provided as a two-part housing having a base part 21 and a lid part 22, and the housing comprises a mounting portion 211 and a carrier portion 212, all as described in connection with FIG. 3.

The insertion module 2 of FIGs. 7A-B differs from the example of FIG. 3 in that the sample container 23 has fluidic interfaces 236 positioned at both ends of the elongated, flat base 230. The base 230 of the sample container comprises the culture chamber, as will be described in more detail below. The fluidic interfaces 236 define one or more inlet and/or outlet ports, which are in fluid communication the culture chamber. As in the previous example, the inlet and/or outlet ports allow the sample container 23 to be fluidly connected to one or more fluid lines for supplying fluid to the culture chamber and for transporting fluid away from the culture chamber, respectively, e.g. for transporting between the sample container and one or more external fluid reservoirs, pumps, fluidic system and/or the like. The base 230 is generally planar and defines a primary plane of the culture chamber.

The insertion module 2 of FIGs. 7A-B further differs from the example of FIG. 3 in that the insertion module of the present example only includes a single radiofrequency resonator 24, which in this example is formed as a single planar coil with multiple turns. The planar coil is arranged adjacent and parallel to one of the walls of the sample container. As in the previous examples, the radiofrequency resonator 24 is configured to transmit a time-varying electromagnetic wave into the sample volume of the culture chamber and/or to detect a time-varying electromagnetic wave from the sample volume of the culture chamber.

## EXAMPLE

Chip creation:

A sample container as illustrated in FIG.s 4 B) and 5A-B, in the following also referred to as fluidic chip, was fabricated using the following materials and procedures: A 1 mm sheet of PETG was covered with a 0.75 mm sheet of SEBS (Eden-Microfluidics, Flexym™), and a 0.3 mm sheet of PETG was covered with a 0.25 mm sheet of SEBS. These composite sheets were cut using a FLUX BeamboxTM $CO_2$ laser operated through Beam StudiosTM software. The laser's intensity, power, and speed were optimized to cut through the SEBS layer without penetrating the PETG layer, allowing for precise removal of the channel shape with tweezers. Subsequently, the chip shape was cut from the PETG layers.

This design enables customization of channel shapes to suit various experimental needs. After cutting the PETG slices, the SEBS was adhered to a sheet of track-etched polycarbonate (ipCELLCULTURE™ Track-Etched Membrane Filter with an 8 $\mu$m pore diameter, a pore density $1\times10^5$/cm pore density, a thickness of 18 $\mu$m, and a porosity of 5%). The membrane was trimmed to fit using a scalpel, and the other PETG slice was pressed on the opposite side, forming a sandwich configuration as shown in FIG. 5A. The assembled chip was then pressed at 2 Pa and 60°C for 1 hour using a hydraulic press.

The fluidic body of the chip was designed using Fusion 360 software to connect to mini-Luer connectors.

As illustrated in FIGs. 5A-B, the design included features for insertion into holes in the top PETG layer, with space allocated for adhesive, The fluidic body was printed using a FormLabs 4B printer with BioMed Clear Resin. Post-printing, the fluidic body was washed with DMSO and cured in a UV box at 70°C for 20 minutes. After curing, the fluidic body was trimmed, autoclaved in 1 L of water, and affixed to the chip using generic superglue.

Chip Treatment and Sterilization:

To ensure minimal leeching from the adhesives, the chip was injected with sterile $H_2O$ and placed in a water-bath for 24 hours. Silicone tubes were attached to the chip with mini Luer connectors, and 70% ETOH was perfused for 1 hr at $500\mu l/min$ using a peristaltic pump, as described in Tan, H. Y. et al. A multi-chamber microfluidic intestinal barrier model using Caco-2 cells for drug transport studies. PLoS One 13, (2018).

Following that, sterile water was flushed into the entire system for an additional 2 hours.

Prior to seeding, the chip was coated using a solution containing 300 $\mu g/mL$ Collagen I solution (Rat Tail Collagen Type I, Gibco) in DMEM, and placed at 37 °C for 1 hr.

Cell Culturing:

The HeLa human cervical cancer cell line (ATCC CCL-2, American Type Culture Collection, Manassas, VA) was regularly cultured in Dulbecco's Modified Eagle Medium (DMEM; Biowest L0103), which contains 4.5 g/L d-glucose, stable I-glutamine, and sodium pyruvate. The medium was supplemented with 10% fetal bovine serum (Biowest S1810) and 1% penicillin/streptomycin (Biowest L0022). Cells were maintained at 37°C in a 5% $CO_2$ environment. The cell medium was refreshed 2 to 3 times per week, and the HeLa cells were cultured until they reached 80% confluence, at which point they were harvested using a 0.25% Trypsin-EDTA solution (Biowest X0930). For the dDNP-NMR experiments, 1 million HeLa cells per T175 flask were seeded 4-5 days prior to the experiment. The cells were harvested by trypsinization, followed by centrifugation and washing in DMEM containing 10 % FBS. Subsequently, the cells were washed in PBS with $Ca^{2+}/Mg^{2+}$ (HyClone, SH30264.01) and resuspended in DMEM to a final concentration of $4 \times 10^6$ cells/mL.

Seeding:

The membrane surface area is 13 $cm^2$ so chips were seeded at a density of $2.5 \times 10^5$ cells/$cm^2$ to a total of $3 \times 10^6$ cells per chip. Chips were left at 37°C, 5% $CO_2$ for three hours before cells were visually adherent. Subsequently, flow was initiated at a flowrate of 150-200 $\mu l/minute$. Media was circulated until the dDNP NMR experiment was set to be conducted.

dDNP-NMR Experiments:

At the dedicated time points, chips were removed from the $CO_2$ incubator and fitted with an injection line at one input port and an exit line at one outlet port. Then the chip was placed into the NMR probe. The probe was subsequently inserted into a Bruker 400 MHz AVANCE NEO spectrometer. Just before injection of the hyperpolarized substrate, the growth medium was removed from the chip using a syringe attached to the injection line. Substrate sample stock solutions were prepared using [1-$^{13}$C] pyruvic acid. The [1-$^{13}$C] pyruvic acid (Sigma-Aldrich) was doped with 17 mM trityl radical AH111501 (GE Healthcare) and 1.5 mM Gadoteridol gadolinium chelate solution (Bracco Imaging).

Approximately 4.5 mg of the pyruvic acid stock was hyperpolarized using a Hypersense 3.3 T polarizer (Oxford Instruments) or (SpinAligner 6.7 T polarizer from Polarize™) until equilibrium polarization was reached, typically within 1 hour. The sample was then quickly dissolved in 5 mL of phosphate buffer (pH 7.4, 40 mM). To stabilize the pH, 5 $\mu$L of 10 M sodium hydroxide solution was added. The final temperature of the solution was maintained at approximately 310 °K.

After dissolution, the concentration of pyruvate was approximately 7 mM. The hyperpolarized substrate solution, which achieved a liquid state polarization of approximately 25%, was collected at the polarizer outlet. Then, 3mL of the solution was drawn into a 5 mL syringe, and 1.5 mL was injected into the chip sitting in the NMR spectrometer through the injection line.

NMR spectra were acquired on a Bruker 400 MHz AVANCE NEO spectrometer. Following injection, a series of $^{13}$C NMR spectra was recorded using a 10° pulse angle with a 2 s delay between pulses.

Counting

Following dDNP NMR, cells were washed twice with PBS, followed by trypsinization for 10 minutes. The outflow was mixed with equal parts DMEM+10% FBS to neutralize the trypsine and spun down before resuspending in 10 mL PBS. Cells were counted using a Countess™ cell counter (Thermo Scientific) with 0.4% trypan blue stain.

Data Analysis

The NMR data analysis was performed using MestReNova software (Mestrelab Research). After phase and baseline correction, the signals of the substrate ([1-$^{13}$C]pyruvate) and the product ([1-$^{13}$C]lactate) in the time series were integrated. A Python-based model was then applied to the integrals using a numerical solution of the ordinary differential equations (ODEs) that describe the forward reaction. In this model, the pulse length and power were fixed, while the T1 values were allowed to vary. The ODEs used in the model are as follows:

$$\frac{dS}{dt} = -k \cdot S(t) - \frac{1}{T1_s} \cdot S(t) - (1 - \cos(pw)^n) \cdot S(t)$$

$$\frac{dP}{dt} = -k \cdot S(t) - \frac{1}{T1_p} \cdot P(t) - (1 - \cos(pw)^n) \cdot P(t)$$

with pw referring to pulse width.

## Coil Design and Functionality

The custom NMR coil integrated with the microfluidic chip was successfully designed and fabricated, as detailed above. The performance of the coil was validated by assessing the signal decay of a bolus injection of hyperpolarized [1-$^{13}$C] pyruvate as illustrated in FIG. 8. In particular, FIG. 8 shows the substrate integral over time (n=3) and comparative substrate integrals (AUC: 24.64 $\pm$ 0.62)

Using the above example of the insertion module, the conversion of hyperpolarized [1-$^{13}$C] pyruvate to [1-$^{13}$C] lactate in HeLa cells was monitored.

## Temporal Analysis of Lactate Production

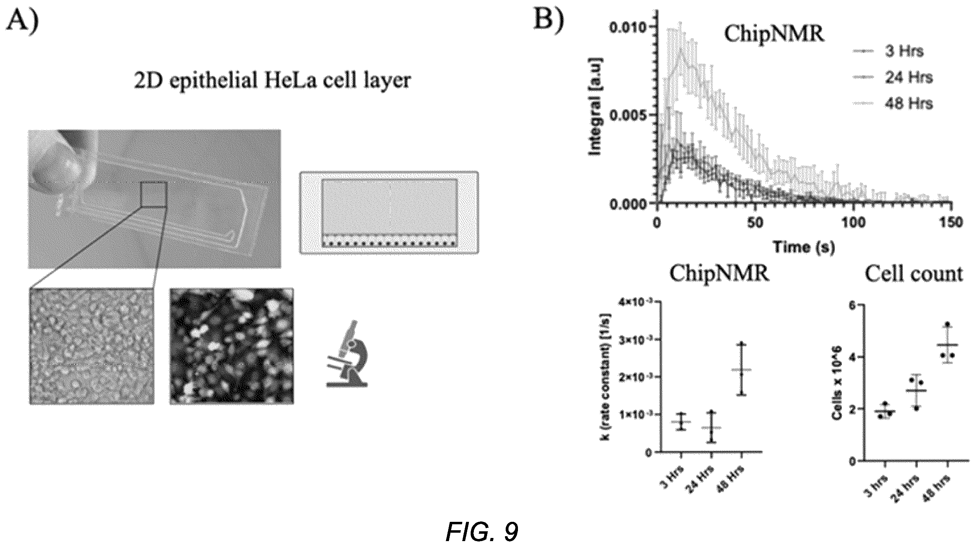

Lactate production was measured at three time points: 3 hours, 24 hours, and 48 hours post HeLa cell seeding. The results, as illustrated in FIG. 9, reveal a steady increase in lactate over time, confirming the system's capability for non-invasive monitoring of cellular metabolism. In particular, FIG. 9 A) shows Brightfield and fluorescence microscopy images (Calcein and Dapi staining) of a confluent layer of HeLa cells three hours after seeding. FIG. 9 B) shows dDNP-NMR measurements of lactate production in a confluent layer of HeLa cells 3, 24, and 48 hours after seeding into the chip (mean live cell count 2 mil, 2.5 mil, 4.5 mil) (Mean +- SD). The increase in kinetic rates seen in FIG. 9 corresponds with the doubling of cell numbers and increase in metabolic viability expected after 48 hours in perfused culture.

HeLa cell viability within the microfluidic chip was maintained throughout the 48-hour experimental period. Post-experiment cell counts indicated consistent cell numbers, suggesting that the perfusion system effectively supported cell survival and stability. The cell count doubled after 48 hours, indicating efficient cell proliferation and survivability within the system.

## Chip with two-compartment chamber for versatile organ-on-chip technology

Embodiments having a two-compartment chamber design enhance the physiological relevance and versatility of organ-on-chip models which expands the functionality and accommodate more complex experimental setups. Using the same ground print, the use of the metabolic measurements was expanded to encompass also chip with a two-compartment chamber, and similar results could be obtained on a confluent epithelial HeLa cell layer, as illustrated in

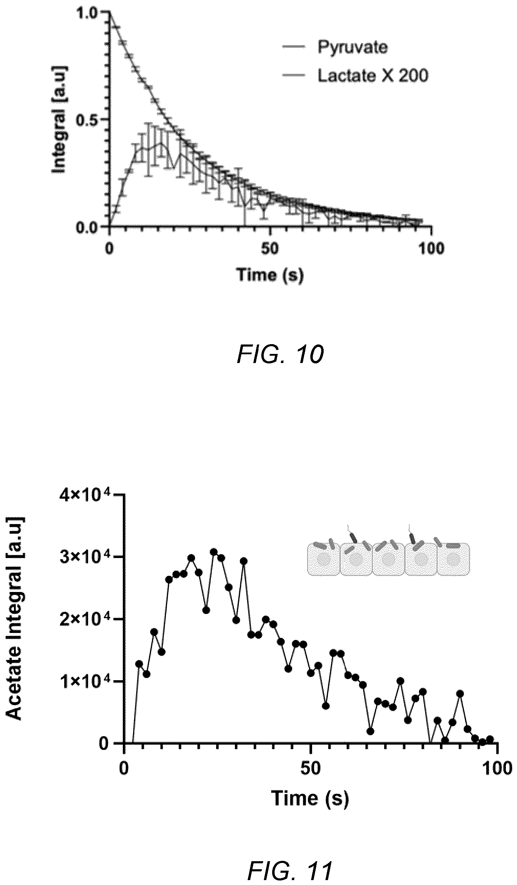

FIG. 10. FIG. 10 shows dDNP-NMR measurement of lactate production in a confluent layer of HeLa cells in a two-compartment chamber three hours after seeding (n=2). No difference in cell growth, metabolism and viability for HeLa cells was observed in the chips with one- and two-compartment chambers.

## Bacterial metabolism from host-bacterial co-culture

In addition to metabolism in a confluent adhering epithelial cell layer (HeLa) we also investigated acetate production following the introduction of a bacterial pathogen (Shigella) causing bloody diarrhea in humans. While Hela metabolizes pyruvate into lactate Shigella produces acetate via the acetate fermentation pathway. The acetate production was monitored over a 180-second period, with measurements taken every 2 seconds. FIG. 11 illustrates the acetate extraction data, where the x-axis represents time in seconds and the y-axis represents the signal intensity (A.U.). The measurements verify the sensitivity of our system, and its potential for use infection metabolism studies.

The above results illustrate that various embodiments of the apparatus disclosed herein provide an efficient integration of NMR technology with organ-on-a-chip systems, providing a robust platform for continuous, noninvasive metabolic monitoring.

One of the major challenges in in vitro studies is replicating the physiological conditions of the human body. The perfusion system within various embodiments disclosed herein more closely mimics in vivo conditions, providing continuous nutrient supply and waste removal. This not only maintains cell viability over extended periods but also ensures that metabolic activity remains consistent and physiologically relevant. The ability to conduct longitudinal studies on the same sample without interruption or loss of viability is a significant advancement, offering deeper insights into long-term metabolic dynamics and cellular responses.

Embodiments disclosed herein are useful in various aspects of preclinical research:

- Drug Development: The apparatus can be used to monitor the metabolic effects of new drug candidates in real time, providing early insights into efficacy and toxicity.

- Personalized Medicine: By studying patient-derived cells, the apparatus may help tailor treatments based on individual metabolic profiles, improving therapeutic outcomes.

- Disease Modeling: The ability to observe metabolic changes in disease states over time offers valuable information for understanding disease progression and identifying potential intervention points.

Understanding the metabolic shifts that occur during host-pathogen interactions is crucial for developing new therapeutic strategies. Traditional methods for metabolic analysis often require the detachment of cells, disrupting cell morphology and adherence, leading to artificial influence on cell metabolism. For example, HeLa cells change from an elongated to a spherical shape post-trypsinization, which can cause adherent bacteria to detach. Embodiments of the apparatus disclosed herein, however, enable direct measurement of metabolic interactions without disrupting host cell morphology. Real-time monitoring of acetate production offers valuable insights into the metabolic demands and adaptations of bacteria under infectious conditions. This information is instrumental in identifying metabolic vulnerabilities and potential therapeutic targets.

As illustrated by the above examples, embodiments of the insertion module disclosed herein allow for exchangeable and highly customizable channel sizes, thicknesses, layers, and components. This flexibility enables the insertion of an extra channel or other experimental necessities, facilitating more complex experimental options

Various embodiments disclosed herein provide a solution that allows for non-invasive metabolic longitudinal studies on microscope compatible perfused sample containers.

Various aspects have been described with reference to various embodiments. Modifications and alterations will occur to others upon reading the present disclosure. It is intended that the invention be construed as including all such modifications and alterations, including insofar as they come within the scope of the appended claims and the equivalents thereof.

**Claims**

1. A culture apparatus for performing nuclear magnetic resonance spectroscopy, the apparatus comprising an insertion module configured to be inserted into a magnet of a system for performing nuclear magnetic resonance spectroscopy, wherein the insertion module comprises:

    - a sample container defining a culture chamber, the culture chamber having a sample volume and being configured to contain a sample of living cells, the culture chamber being in fluidic connection with at least one supply channel for providing a fluid flow through the culture chamber,
    - one or more radiofrequency resonators, each configured to transmit a time-varying electromagnetic wave into the sample volume of the culture chamber and/or to detect a time-varying electromagnetic wave from the sample volume of the culture chamber,

    wherein the sample container comprises a cell support structure defining at least one cell adherence surface configured to carry a culture of living cells within the culture chamber.

2. The culture apparatus according to claim 1, wherein each of the one or more radiofrequency resonators is selected from the group of radiofrequency resonators consisting of: a parallel plate resonator, a surface coil with a single turn, a surface coil with a plurality of turns, a saddle coil.

3. The culture apparatus according to claim 2, wherein the surface coil is a planar coil.

4. The culture apparatus according to any one of the preceding claims, wherein the culture chamber is a flat chamber having a width and a length defining a primary plane of the culture chamber, wherein the culture chamber has a height smaller than the width and the length, wherein the radiofrequency resonator defines a resonator plane substantially parallel to the primary plane.

5. The culture apparatus according to claim 4, wherein the radiofrequency resonator has a resonator width and a resonator length, wherein the resonator width is no smaller than the width of the culture chamber, and wherein the resonator length is no smaller than the length of the culture chamber.

6. The culture apparatus according to claim 4 or 5, wherein the at least one cell adherence surface extends parallel to the primary plane of the culture chamber.

7. The culture apparatus according to any one of the preceding claims, wherein the radiofrequency resonator is arranged adjacent the sample container and configured to concentrate the transmitted electromagnetic wave inside the culture chamber.

8. The culture apparatus according to any one of the preceding claims, wherein the radiofrequency resonator has two resonator portions arranged adjacent opposite sides of the culture chamber.

9. The culture apparatus according to any one of the preceding claims, comprising at least two radiofrequency resonators configured to be resonant at respective resonant frequencies.

10. The culture apparatus according to any one of the preceding claims, wherein the cell support structure comprises a membrane, in particular a track-etched polycarbonate membrane, suspended within the culture chamber.

11. The culture apparatus according to any one of the preceding claims, wherein the cell adherence surface defines a cell adherence surface area of more than 20 mm$^2$, preferably more than 1000 mm$^2$.

12. The culture apparatus according to any one of the preceding claims, wherein the cell support structure is suspended so as to divide the culture chamber into two compartments, wherein the cell support structure defines a first cell adherence surface facing a first compartment of the two compartments.

13. The culture apparatus according to any one of the preceding claims, wherein the sample container is an elongated container having a first end and a second end, opposite the first end, and wherein the sample container comprises an inlet port and an outlet port, each in fluid communication with the culture chamber, wherein the inlet port and the outlet port are arranged at the first end.

14. The culture apparatus according to any one of the preceding claims, wherein the supply channel has a divergent inlet portion providing an inlet opening for providing fluid flow from the supply channel into the culture chamber, the inlet opening having a cross-sectional area larger than a cross-sectional area of the supply channel.

15. The culture apparatus according to any one of the preceding claims, wherein the sample volume of the culture chamber is at least 200 μl, such as at least 500 μl, such as at least 700 μl.

FIG. 1

FIG. 2

FIG. 3

230    238    230
2381
237    237
238
236    236
2361    A)    B)    2361

*FIG. 4*

231
232
237
233
234
235

231    239    239
239    239
238
232
2321
234
233    235
230

*FIG. 5A*

2361    2361    2361
2361    2361
236
2362    2362
2362    2362

*FIG. 5B*

237    233

2371

2372

*FIG. 6A*

237

233

*FIG. 6B*

Pyruvate

Pyruvate

*FIG. 8*

FIG. 7A

FIG. 7B

FIG. 9

*FIG. 10*

*FIG. 11*

Europäisches Patentamt
European Patent Office
Office européen des brevets

# EUROPEAN SEARCH REPORT

**Application Number**

EP 25 15 4601

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 431 965 A1 (FUNDACIO INST DE BIOENGINYERIA DE CATALUNYA [ES] ET AL.) 18 September 2024 (2024-09-18) | 1-9,11, 13,15 | INV.<br>G01N24/08<br>G01R33/30 |
| Y | * paragraphs [0053], [0055], [0064], [0069], [0072], [0073]; figures 2,3 *<br>----- | 1,4,5 | G01R33/465<br>C12M3/06 |
| Y | WO 2024/089178 A1 (FUNDACIO INST DE BIOENGINYERIA DE CATALUNYA [ES]; VITALA TECH S L [ES]) 2 May 2024 (2024-05-02)<br>* paragraph [0071]; figure 3d *<br>----- | 1,4,5 | ADD.<br>G01R33/34<br>G01R33/36<br>G01R33/46 |
| A,D | WO 2023/111559 A1 (UNIV SOUTHAMPTON [GB]) 22 June 2023 (2023-06-22)<br>* claim 1; figure 10 *<br>----- | 1-15 | |
| A | MANGAS-FLORENCIO LLUÍS ET AL: "A DIY Bioreactor for in Situ Metabolic Tracking in 3D Cell Models via Hyperpolarized 13 C NMR Spectroscopy",<br>ANALYTICAL CHEMISTRY, [Online]<br>vol. 97, no. 3,<br>15 January 2025 (2025-01-15), pages 1594-1602, XP093288467,<br>ISSN: 0003-2700, DOI:<br>10.1021/acs.analchem.4c04183<br>Internet<br>Retrieved from the Internet:<br>URL:https://pubs.acs.org/doi/pdf/10.1021/acs.analchem.4c04183><br>[retrieved on 2025-06-20]<br>* the whole document *<br>----- | 1 | |
| A | US 10 436 726 B2 (UNIV MACAU [CN]) 8 October 2019 (2019-10-08)<br>* figure 9 *<br>----- | 1-5 | |

**TECHNICAL FIELDS SEARCHED (IPC)**

G01N
G01R
C12M

The present search report has been drawn up for all claims

| Place of search | Date of completion of the search | Examiner |
|---|---|---|
| Munich | 25 June 2025 | Skalla, Jörg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

...........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 15 4601

This annex lists the patent family members relating to the patent documents cited in the above-mentioned European search report.
The members are as contained in the European Patent Office EDP file on
The European Patent Office is in no way liable for these particulars which are merely given for the purpose of information.

25-06-2025

| Patent document cited in search report | | Publication date | Patent family member(s) | | Publication date |
|---|---|---|---|---|---|
| EP 4431965 | A1 | 18-09-2024 | EP | 4431965 A1 | 18-09-2024 |
| | | | WO | 2024189126 A1 | 19-09-2024 |
| WO 2024089178 | A1 | 02-05-2024 | NONE | | |
| WO 2023111559 | A1 | 22-06-2023 | NONE | | |
| US 10436726 | B2 | 08-10-2019 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5517856 A

- WO 2023111559 A

**Non-patent literature cited in the description**

- **TAN, H. Y. et al.** A multi-chamber microfluidic intestinal barrier model using Caco-2 cells for drug transport studies. *PLoS One*, 2018, vol. 13